(19) Europäisches Patentamt
European Patent Office
Office européen des brevets

(11) **EP 4 432 699 A1**

(12) **EUROPEAN PATENT APPLICATION**
published in accordance with Art. 153(4) EPC

(43) Date of publication:
**18.09.2024 Bulletin 2024/38**

(21) Application number: **23750005.3**

(22) Date of filing: **06.02.2023**

(51) International Patent Classification (IPC):
*H04R 1/10* (2006.01)   *H05B 33/12* (2006.01)
*G02F 1/15* (2019.01)   *G01K 11/12* (2021.01)
*G01J 5/60* (2006.01)   *A61B 5/024* (2006.01)
*A61B 5/1455* (2006.01)   *A61B 5/145* (2006.01)
*A61B 5/00* (2006.01)

(52) Cooperative Patent Classification (CPC):
**A61B 5/00; A61B 5/024; A61B 5/145;
A61B 5/1455; G01J 5/60; G01K 11/12; G02F 1/15;
H04R 1/10; H05B 33/12**

(86) International application number:
**PCT/KR2023/001674**

(87) International publication number:
**WO 2023/149772 (10.08.2023 Gazette 2023/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.02.2022 KR 20220015367**

(71) Applicant: **Samsung Electronics Co., Ltd.
Suwon-si, Gyeonggi-do 16677 (KR)**

(72) Inventor: **SHCHUR, Oleksandr
Kyiv, 01032 (UA)**

(74) Representative: **Appleyard Lees IP LLP
15 Clare Road
Halifax HX1 2HY (GB)**

(54) **ELECTRONIC DEVICE COMPRISING CHROMIC MEMBER**

(57)     An electronic device is provided. The electronic device includes a housing including a protrusion, an ear tip configured to be connected to the protrusion, a discoloration member disposed on an inner surface of the ear tip and configured to change hue based on temperature, a light emitting module disposed in the housing and configured to emit light toward the discoloration member, a first optical sensor disposed in the housing and configured to detect light reflected from the discoloration member, and a processor configured to determine a user's temperature based on a hue of light detected by the first optical sensor.

FIG. 5

**Description**

[Technical Field]

The disclosure relates to an electronic device including a discoloration member. More particularly, the disclosure relates to a wearable electronic device including a discoloration member that changes a hue based on temperature.

[Background Art]

With the development of information communication technology and semiconductor technology, various functions have come to be incorporated into a single portable electronic device. For example, an electronic device may implement entertainment functions such as gaming, multimedia functions such as music/video playback, communication and security functions for mobile banking, and schedule management or electronic wallet functions, in addition to communication functions. Such an electronic device becomes increasingly more compact so that the user can conveniently carry it. Particularly, the electronic device has been reduced in size and weight so that the electronic device can be used without any inconvenience even in the state in which the electronic device is worn on a body.

The above information is presented as background information only to assist with an understanding of the disclosure. No determination has been made, and no assertion is made, as to whether any of the above might be applicable as prior art with regard to the disclosure.

[Disclosure]

[Technical Problem]

An electronic device that is wearable on a body may include components related to at least one or more sound effects. For example, a wearable electronic device including a speaker and a microphone may be worn on a portion close to a user's ear, such as an in-ear earphone (or an earset) or a hearing aid.

The wearable electronic device may detect a user's body temperature in the user's ear. The temperature detected in the user's ear may be closer to a core temperature of the user than a temperature of other parts of the user's body (e.g., a wrist or a forehand). For example, the wearable electronic device may detect the user's temperature using a temperature sensor disposed on an ear tip. When the temperature sensor is disposed on the ear tip, however, there may be a resultant increase in breakage of the temperature sensor.

Aspects of the disclosure are to address at least the above-mentioned problems and/or disadvantages and to provide at least the advantages described below. Accordingly, an aspect of the disclosure is to provide an electronic device capable of determining a user's core temperature using a discoloration member disposed on an ear tip, and an optical sensor configured to detect a hue of the discoloration member.

Another aspect of the disclosure is to provide an electronic device capable of determining the user's biometric information.

However, the problem to be solved in the disclosure is not limited to the above-mentioned problem, and may be variously expanded without departing from the spirit and scope of the disclosure.

Additional aspects of the disclosure will be set forth in part in the description which follows and, in part, will be apparent from the description, or may be learned by practice of the presented embodiments.

[Technical Solution]

In accordance with an aspect of the disclosure, an electronic device is provided. The electronic device may include a housing including a protrusion, an ear tip configured to be connected to the protrusion, a discoloration member disposed on an inner surface of the ear tip and configured to change hue based on temperature, a light emitting module disposed in the housing and configured to emit light toward the discoloration member, a first optical sensor disposed in the housing and configured to detect light reflected from the discoloration member, and a processor configured to determine a user's temperature based on a hue of light detected by the first optical sensor.

In accordance with another aspect of the disclosure, an electronic device is provided. The electronic device may include a housing, an ear tip configured to be connected to the housing, a discoloration member disposed on the ear tip and configured to change a hue based on temperature, a light emitting module disposed in the housing and configured to emit light toward the discoloration member and a user's body, a first optical sensor disposed in the housing and configured to detect light reflected from the discoloration member, and a second optical sensor disposed in the housing and configured to detect light reflected from the user's body, and a processor configured to determine the user's temperature based on a hue of light detected by the first optical sensor, and to determine the user's biometric information based on a signal detected using the second optical sensor.

[Advantageous Effects]

According to an embodiment of the disclosure, the electronic device may measure the user's body temperature in the user's ear. As the user's body temperature is measured in the ear, the user's core temperature having less influence from an external environment can be measured, and the accuracy of body temperature measurement may increase.

According to an embodiment of the disclosure, the electronic device may determine the user's body temperature by detecting the hue of the discoloration member configured to change a hue based on the user's body temperature. As the hue of the discoloration member is detected using the optical sensor, no sensor is disposed on the ear tip, thereby increasing durability of the electronic device.

According to an embodiment of the disclosure, the electronic device may detect the user's biometric information and determine the user's health index, by detecting the light generated by the light emitting part using the optical sensor.

Other aspects, advantages, and salient features of the disclosure will become apparent to those skilled in the art from the following detailed description, which, taken in conjunction with the annexed drawings, discloses various embodiments of the disclosure.

[Description of Drawings]

The above and other aspects, features, and advantages of an embodiment of the disclosure will be more apparent from the following description taken in conjunction with the accompanying drawings, in which:

FIG. 1 is a block diagram of an electronic device in a network environment according to an embodiment of the disclosure;

FIG. 2 is a block diagram of an audio module according to an embodiment of the disclosure;

FIG. 3 is a perspective view of an electronic device according to an embodiment of the disclosure;

FIG. 4 is a schematic view of an electronic device including a first optical sensor according to an embodiment of the disclosure;

FIG. 5 is a view of an electronic device including a first optical sensor and a second optical sensor according to an embodiment of the disclosure;

FIG. 6 is a perspective view of an electronic device including a sensor module according to an embodiment of the disclosure;

FIG. 7 is a block diagram illustrating an operation of an electronic device according to an embodiment of the disclosure;

FIG. 8 is a view illustrating a change in a hue of a discoloration member that is changed based on a second light detected by the first optical sensor according to an embodiment of the disclosure;

FIG. 9 is a view illustrating a value of photoplethysmography (PPG) that is changed based on a fourth light detected by the second optical sensor according to an embodiment of the disclosure;

FIG. 10 is a block diagram illustrating an operation of an electronic device according to an embodiment of the disclosure; and

FIG. 11 is a flowchart illustrating an operation of an electronic device according to an embodiment of the disclosure.

Throughout the drawings, like reference numerals will be understood to refer to like parts, components, and structures.

[Mode for Invention]

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

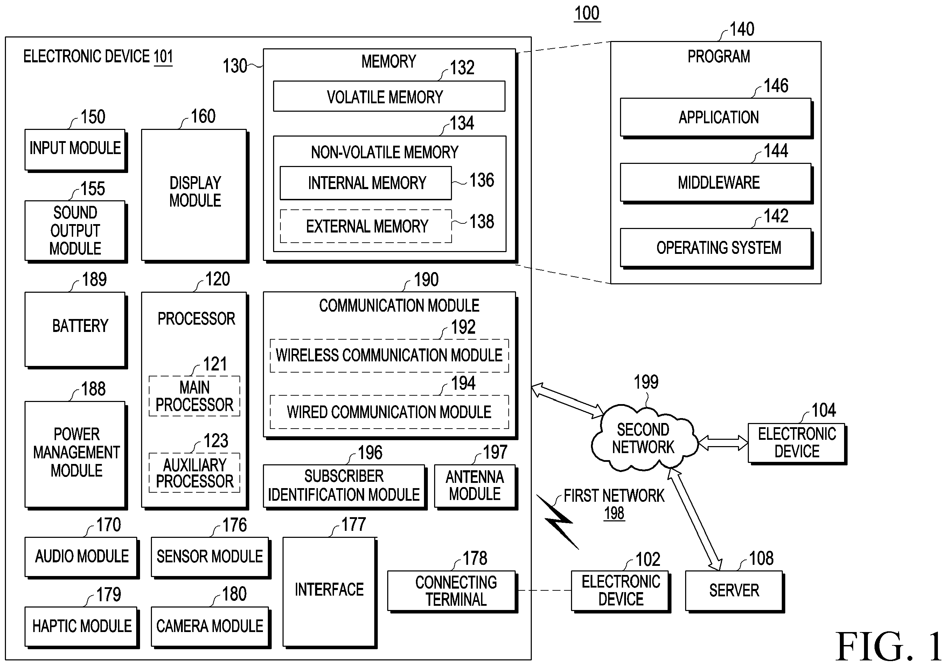

FIG. 1 is a block diagram illustrating an electronic device in a network environment according to an embodiment of the disclosure.

Referring to FIG. 1, the electronic device 101 in the network environment 100 may communicate with an external electronic device 102 via a first network 198 (e.g., a short-range wireless communication network), or an external electronic device 104 or a server 108 via a second network 199 (e.g., a long-range wireless communication network). According to an embodiment, the electronic device 101 may communicate with the external electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134. The non-volatile memory 134 may include an internal memory 136 and/or an external memory 138.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display 160 may include, for example, a display, a hologram device, or a projector and control circuitry to control a corresponding one of the display, hologram device, and projector. According to an embodiment, the display 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an external electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the external electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the external electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the external electronic device 102, the external electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth™, Wi-Fi direct, or infrared data association (IrDA)) or a second network 199 (e.g., a long-range communication network, such as a legacy cellular network, a 5$^{th}$ generation (5G) network, a next-generation communication network, the Internet, or a computer network (e.g., local area network (LAN) or wide area network (WAN))). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4$^{th}$ generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beam-forming, massive multiple-input and multiple-output (massive MIMO), full dimensional MIMO (FD-MIMO), array antenna, analog beam-forming, or large-scale antenna. The wireless communication module 192 may support various requirements specified in the electronic device 101, an external electronic device (e.g., the external electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra-low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or health-care) based on 5G communication technology or IoT-related technology.

An electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively," as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an embodiment, the module may be implemented in a form of an application-specific integrated circuit (ASIC).

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

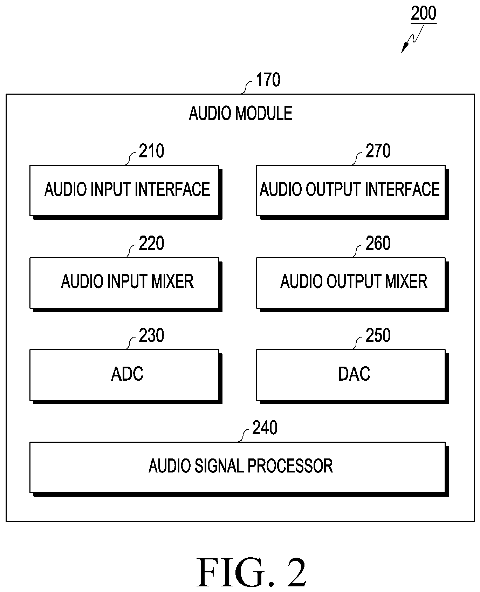

FIG. 2 is a block diagram 200 illustrating the audio module 170 according to an embodiment of the disclosure. Referring to FIG. 2, the audio module 170 may include, for example, an audio input interface 210, an audio input mixer 220, an analog-to-digital converter (ADC) 230, an audio signal processor 240, a digital-to-analog converter (DAC) 250, an audio output mixer 260, or an audio output interface 270.

The audio input interface 210 may receive an audio signal corresponding to a sound obtained from the outside of the electronic device 101 via a microphone (e.g., a dynamic microphone, a condenser microphone, or a piezo microphone) that is configured as part of the input device 150 or separately from the electronic device 101. For example, if an audio signal is obtained from the external electronic device 102 (e.g., a headset or a microphone), the audio input interface 210 may be connected with the external electronic device 102 directly via the connecting terminal 178, or wirelessly (e.g., BluetoothTM communication) via the wireless communication module 192 to receive the audio signal. According to an embodiment, the audio input interface 210 may receive a control signal (e.g., a volume adjustment signal received via an input button) related to the audio signal obtained from the external electronic device 102. The audio input interface 210 may include a plurality of audio input channels and may receive a different audio signal via a corresponding one of the plurality of audio input channels, respectively. According to an embodiment, additionally or alternatively, the audio input interface 210 may receive an audio signal from another component (e.g., the processor 120 or the memory 130) of the electronic device 101.

The audio input mixer 220 may synthesize a plurality of inputted audio signals into at least one audio signal. For example, according to an embodiment, the audio input mixer 220 may synthesize a plurality of analog audio signals inputted via the audio input interface 210 into at least one analog audio signal.

The ADC 230 may convert an analog audio signal into a digital audio signal. For example, according to an embodiment, the ADC 230 may convert an analog audio signal received via the audio input interface 210 or, additionally or alternatively, an analog audio signal synthesized via the audio input mixer 220 into a digital audio signal.

The audio signal processor 240 may perform various processing on a digital audio signal received via the ADC 230 or a digital audio signal received from another component of the electronic device 101. For example, according to an embodiment, the audio signal processor 240 may perform changing a sampling rate, applying one or more filters, interpolation processing, amplifying or attenuating a whole or partial frequency bandwidth, noise processing (e.g., attenuating noise or echoes), changing channels (e.g., switching between mono and stereo), mixing, or extracting a specified signal for one or more digital audio signals. According to an embodiment, one or more functions of the audio signal processor 240 may be implemented in the form of an equalizer.

The DAC 250 may convert a digital audio signal into an analog audio signal. For example, according to an embodiment, the DAC 250 may convert a digital audio signal processed by the audio signal processor 240 or a digital audio signal obtained from another component (e.g., the processor 120 or the memory 130) of the electronic device 101 into an analog audio signal.

The audio output mixer 260 may synthesize a plurality of audio signals, which are to be outputted, into at least one audio signal. For example, according to an embodiment, the audio output mixer 260 may synthesize an analog audio signal converted by the DAC 250 and another analog audio signal (e.g., an analog audio signal received via the audio input interface 210) into at least one analog audio signal.

The audio output interface 270 may output an analog audio signal converted by the DAC 250 or, additionally or alternatively, an analog audio signal synthesized by the audio output mixer 260 to the outside of the electronic device 101 via the sound output device 155. The sound output device 155 may include, for example, a speaker, such as a dynamic driver or a balanced armature driver, or a receiver. According to an embodiment, the sound output device 155 may include a plurality of speakers. In such a case, the audio output interface 270 may output audio signals having a plurality of different channels (e.g., stereo channels or 5.1 channels) via at least some of the plurality of speakers. According to an embodiment, the audio output interface 270 may be connected with the external electronic device 102 (e.g., an external speaker or a headset) directly via the connecting terminal 178 or wirelessly via the wireless communication module 192 to output an audio signal.

According to an embodiment, the audio module 170 may generate, without separately including the audio input mixer 220 or the audio output mixer 260, at least one digital audio signal by synthesizing a plurality of digital audio signals using at least one function of the audio signal processor 240.

According to an embodiment, the audio module 170 may include an audio amplifier (not shown) (e.g., a speaker amplifying circuit) that is capable of amplifying an analog audio signal inputted via the audio input interface 210 or an audio signal that is to be outputted via the audio output interface 270. According to an embodiment, the audio amplifier may be configured as a module separate from the audio module 170.

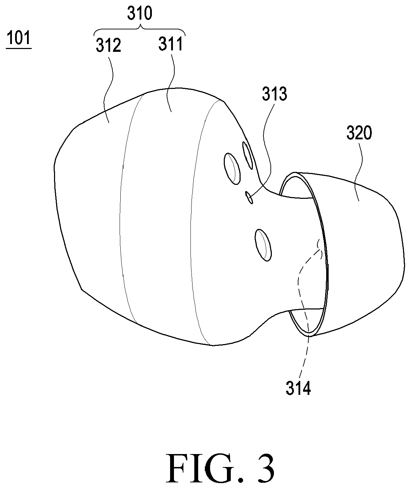

FIG. 3 is a perspective view of an electronic device according to an embodiment of the disclosure.

Referring to FIG. 3, the electronic device 101 may include a housing 310 configured to accommodate components of the electronic device 101. For example, a sound component (e.g., the audio module 170 of FIG. 2) and/or electronic components (e.g., the processor 120, the power management module 188, and the battery 189 of FIG. 1), or a wireless communication module 192 may be disposed in the housing 310. The configurations of the electronic device 101 of FIG. 3 may be substantially the same as all or some of the configurations of the electronic device 101 of FIG. 1.

According to various embodiments, the electronic device 101 may include a wearable electronic device. For example, the electronic device 101 may be worn on a part of a user's body, for example, such as ears or a head. According to an embodiment, the electronic device 101 may include an in-ear earset, an in-ear headset or a hearing aid.

According to various embodiments, the electronic device 101 may be electrically connected to an external electronic device (e.g., the external electronic device 102 of FIG. 1). According to an embodiment, the electronic device 101 may function as an audio output interface (or, for example, the sound output module 155 of FIG. 1) that outputs a sound signal received from the external electronic device 102 to the outside. Additionally or alternatively, the electronic device 101 disclosed in the disclosure may function as an audio input interface (or the input module 150 of FIG. 1) configured to receive an audio signal corresponding to a sound obtained from the outside of the electronic device 101.

According to an embodiment, the electronic device 101 may communicate with the external electronic device 102 or may be controlled by the external electronic device 102. The electronic device 101 may be an interaction-type electronic device that is paired with an external electronic device such as a smart phone through a communication method such as Bluetooth, to convert data received from the external electronic device 102 to output a sound, or to receive a user's voice to transmit the received voice to the external electronic device 102.

According to an embodiment, the electronic device 101 may be wirelessly connected to the external electronic device 102. For example, the electronic device 101 may communicate with the external electronic device 102 through a network (e.g., a short-range wireless communication network or a long-range wireless communication network). The network may include, but is not limited to, a mobile or cellular communication network, a local area network (LAN) (e.g., Bluetooth communication), a wireless local area network (WLAN), a wide area network (WAN), the Internet, or a small area network (SAN). According to an embodiment, the electronic device 101 may be wiredly connected to the external electronic device 102 using a cable (not shown).

According to another embodiment, the electronic device 101 may not communicate with the external electronic device 102. In this case, the electronic device 101 is not controlled by the external electronic device 102, and may be implemented to receive a signal corresponding to a sound obtained from the outside according to operations (or control) of components *per se* included in the electronic device 101, and output the sound signal to the outside. For example, the electronic device 101 may be a stand-alone electronic device that plays music or video back by itself without communicating with the external electronic device 102 to output a corresponding sound or to receive and process a user's voice.

In various drawings of the disclosure, as an example of the electronic device 101, a kernel-type in-ear earset configured to be mounted in an external auditory meatus starting from a pinna to a tympanic membrane will be mainly described. However, it should be noted that the disclosure is not limited thereto. According to another embodiment, although not shown in the drawings, it will be described that the electronic device 101 may be also an open-type earset configured to be mounted on the pinna.

According to various embodiments, the housing 310 may include a plurality of components. For example, the housing 310 may include a first housing 311 and a second housing 312 connected with the first housing 311. According to an embodiment, the first housing 311 and the second housing 312 may form at least a portion of an external appearance of the electronic device 101 and form an inner space in which components of the electronic device 101 may be accommodated. According to an embodiment, when the electronic device 101 is worn on the user, at least a portion of the first housing 311 may contact or face the user's body (e.g., an ear), and at least a portion of the second housing 312 may face an opposite direction to the user.

According to various embodiments, the housing 310 may include a microphone hole 313. According to an embodiment, the microphone hole 313 may be interpreted as a through hole formed in the first housing 311 and/or the second housing 312. According to an embodiment, the external sound of the electronic device 101 may be transmitted to a microphone module (e.g., the audio module 170 of FIG. 2) disposed inside the electronic device 101 via the microphone hole 313. According to an embodiment, the microphone hole 313 may include a plurality of microphone holes.

According to various embodiments, the housing 310 may include a protrusion 314. According to an embodiment, at least a portion of the protrusion 314 may be inserted into the user's body (e.g., an ear). For example, the electronic device 101 may be inserted and mounted in the user's body (e.g., the external auditory meatus or pinna of the user's body) using the protrusion 314. According to an embodiment, the protrusion 314 may be interpreted as a part of the housing 310 extending from the first housing 311. According to an embodiment, the protrusion 314 may be connected to an ear tip 320.

According to an embodiment, the electronic device 101 may be in close contact with the user's ear using the ear tip. According to an embodiment, the protrusion 314 may include at least one recess (not shown), and a sound output from a speaker module (e.g., the audio module 170 of FIG. 2) disposed inside the electronic device 101 may be radiated to the outside of the electronic device 101 using the recess located in the protrusion 314. According to an embodiment, the ear tip 320 may be made of an elastic material. For example, the ear tip 320 may include silicone.

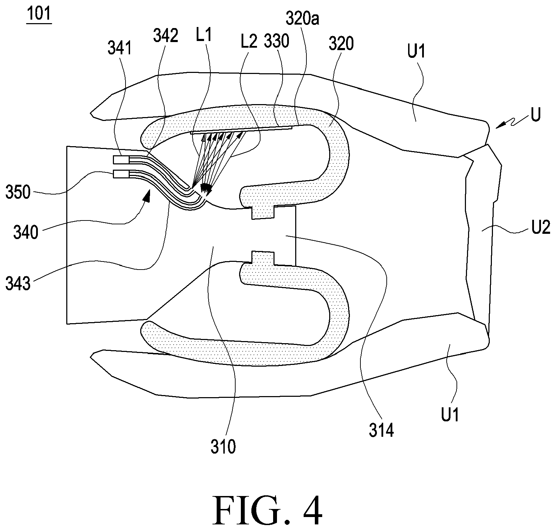

FIG. 4 is a schematic view of an electronic device including a first optical sensor according to an embodiment of the disclosure.

Referring to FIG. 4, the electronic device 101 may include the housing 310 and the ear tip 320. The configurations of the housing 310 and ear tip 320 shown in FIG. 4 may be entirely or partially the same as the configurations of the housing 310 and ear tip 320 shown in FIG. 3.

According to an embodiment, the electronic device 101 may be worn on the body (e.g., ears) of a user U. For example, at least a portion of the ear tip 320 of the electronic device 101 may be inserted into an ear channel U1 of the user U. At least a portion of the ear tip 320 may be in contact with the ear channel U1, and at least a portion of the sound generated by the electronic device 101 may be transmitted to the tympanic membrane of the user U2.

According to an embodiment, the electronic device 101 may include a discoloration member 330 configured to change a hue based on temperature. For example, the hue of the discoloration member 330 may be changed based on the temperature of the user U. For example, as the discoloration member 330 is higher in temperature (e.g., 42°C), the hue of the discoloration member 330 may be changed to blue, and as the discoloration member 330 is lower in temperature (e.g., 34°C), the hue of the discoloration member 330 may be changed to red.

According to an embodiment, the discoloration member 330 may include thermochromic liquid crystals (TLC). For example, the discoloration member 330 may include cholesteryl oleyl carbonate, cholesteryl nonanoate, and cholesteryl benzoate. According to an embodiment, the discoloration member 330 may include the cholesteryl oleyl carbonate, the cholesteryl nonanoate and the cholesteryl benzoate in a ratio of 3:6:1, respectively. According to an embodiment, the hue of the discoloration member 330 may be changed within a designated temperature range (e.g., about 37 to 40 degrees Celsius).

According to an embodiment, since the discoloration member 330 is disposed close to the ear of the user U, the discoloration member 330 may change its own hue based on the user's core temperature. The processor (e.g., the processor 120 of FIG. 1) may determine the user's core temperature, thereby reducing the influence of the external environment and increasing the accuracy of determining the user's body information (e.g., body temperature).

According to an embodiment, the discoloration member 330 may be disposed on an inner surface 320a of the ear tip 320. According to an embodiment, the discoloration member 330 may be coated on the inner surface 320a of the ear tip 320.

According to an embodiment, at least a portion of the discoloration member 330 may be visually exposed to the outside of the electronic device 101. For example, the discoloration member 330 may be visually recognized from the outside of the electronic device 101 via the ear tip 320. According to an embodiment, the discoloration member 330 may receive at least a portion of the light generated by the light emitting module 340.

According to an embodiment, the electronic device 101 may include the light emitting module 340. According to an embodiment, the light emitting module 340 may emit light of at least two or more distinguishable wavelengths. For example, the light emitting module 340 may include a light emitting diode 341. The light emitting diode 341 may emit at least two distinguishable wavelengths. For example, the light emitting diode 341 may include a red light emitting diode, a green light emitting diode, and/or a blue light emitting diode. According to an embodiment, the light emitting module 340 may be disposed in the housing 310.

According to an embodiment, at least a portion (e.g., the first light L1) of the light generated by the light emitting module 340 may be transmitted to the discoloration member 330.

According to an embodiment, the light emitting module 340 may include a first light path 342 for guiding at least a portion of the light generated by the light emitting diode 341 to the discoloration member 330. An end of the first light path 342 may be connected to the light emitting diode 341, and the other end thereof may be disposed to face the discoloration member 330. According to an embodiment, the first light path 342 may include an optical cable and/or a waveguide.

According to an embodiment, the electronic device 101 may include a first optical sensor 350. According to an embodiment, the first optical sensor 350 may detect at least a portion of light (e.g., a second light L2) that is generated by the light emitting module 340 and reflected from the discoloration member 330. According to an embodiment, the first optical sensor 350 may be disposed in the housing 310.

According to an embodiment, the first optical sensor 350 may detect a hue of light reflected from the discoloration member 330.

According to an embodiment, the processor (e.g., the processor 120 of FIG. 1) may determine the temperature of the user U based on the hue detected by the first optical sensor 350. For example, the processor 120 may detect a wavelength of light (e.g., the second light L2) reflected from the discoloration member 330, and determine the temperature of the user U based on the detected wavelength of the light.

According to an embodiment, the light emitting module 340 may include a second light path 343 for guiding at least a portion of light reflected from the discoloration member 330 to the first optical sensor 350. An end of the second light path 342 may be disposed to face the discoloration member 330, and the other end of the second light path 342 may be connected to the first light sensor 350. According to an embodiment, the second light path 343 may include an optical cable and/or a waveguide.

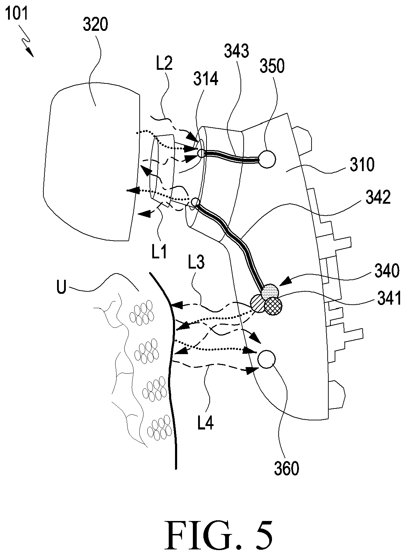

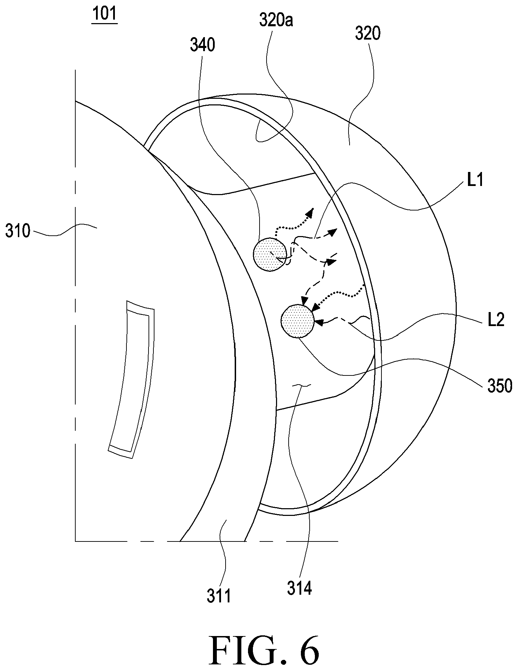

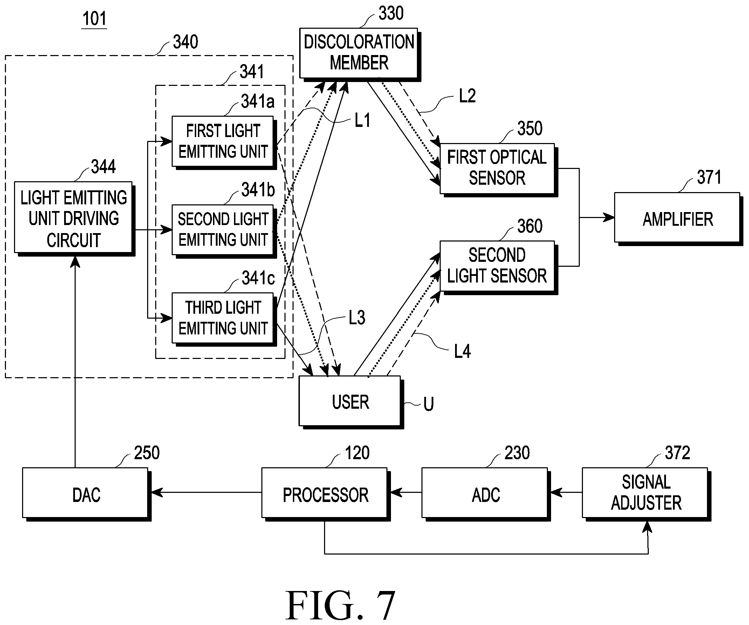

FIG. 5 is a view of an electronic device including a first optical sensor and a second optical sensor, according to an embodiment of the disclosure. FIG. 6 is a perspective view of an electronic device including a sensor module, according to an embodiment of the disclosure. FIG. 7 is a block diagram illustrating an operation of an electronic device according to an embodiment of the disclosure.

Referring to FIGS. 5, 6, and/or 7, the electronic device 101 may include the processor 120, an Analog to Digital Converter (ADC) 230, a Digital to Analog Converter (DAC) 250, the housing 310, the ear tip 320, the discoloration member 330, the light emitting module 340, the first optical sensor 350, and the second optical sensor 360. The configurations of the housing 310, the ear tip 320, the light emitting module 340 and the first optical sensor 350 of FIGS. 5 and/or 6 may be entirely or partially the same as those of the housing 310, the ear tip 320, the light emitting module 340, and the first optical sensor 350 of FIG. 4. The configurations of the processor 120 of FIG. 7 may be entirely or partially the same as those of the processor 120 of FIG. 1, and the configurations of the ADC 230 and the DAC 250 of FIG. 7 may be entirely or partially the same as those of the ADC 230 and the DAC 250 of FIG. 2. The configurations of the discoloration member 330 of FIG. 7 may be entirely or partially the same as those of the discoloration member 330 of FIG. 4.

According to an embodiment, the processor 120 may control the light emitting module 340 using the DAC 250. For example, the processor 120 may control driving a light emitting unit driving circuit 344 configured to control driving the light emitting diode 341. According to an embodiment, the light emitting module 340 may emit light toward the outside (e.g., the body of the user U) of the electronic device 101. According to an embodiment, the first optical sensor 350 and the second optical sensor 360 may use the same light emitting diode 341. For example, a portion of light generated by the light emitting diode 341 may be transmitted to the discoloration member 330 via the first light path 342, and another portion of the light generated by the light emitting diode 341 may be transmitted to the body of the user U. The discoloration member 330 may be disposed on at least a portion of the inner surface 320a of the ear tip 320. According to an embodiment, the light emitting diode 341 may include a plurality of light emitting units for emitting light of different wavelengths. For example, the light emitting diode 341 may include a first light emitting unit 341a for emitting red light, a second light emitting unit 341b for emitting green light, and/or a third light emitting unit 341c for emitting blue light.

According to an embodiment, a first light L1, which is a portion of light generated by the light emitting diode 341, may be reflected from the discoloration member 330. A second light L2 reflected from the discoloration member 330 may be transmitted to the first optical sensor 350 via the second light path 343.

According to an embodiment, a third light L3, which is a portion of light generated by the light emitting diode 341, may be reflected from the body of the user U. A fourth light L4 reflected from the body of the user U may be transmitted to the second optical sensor 360.

According to an embodiment, the first optical sensor 350 may be disposed to be spaced apart from the second optical sensor 360. For example, the first optical sensor 350 may be disposed closer to the protrusion 314 and/or the ear tip 320 than the second optical sensor 360. According to an embodiment, the first optical sensor 350 and the second optical sensor 360 may be disposed in the first housing (e.g., the first housing 311 of FIG. 3).

According to an embodiment, the first optical sensor 350 may be interpreted as a color sensor for detecting the hue of the discoloration member 330. For example, as the first light L1 is reflected from the discoloration member 330, the second light L2 may have a different wavelength from that of the first light L1. According to an embodiment, the processor 120 may determine the hue of the discoloration member 330 based on a signal detected by the first optical sensor 350.

According to an embodiment, the second optical sensor 360 may be referred to as a biometric information detection sensor. For example, the second optical sensor 360 may be interpreted as a sensor for photoplethysmography (PPG) and/or a sensor for saturation of percutaneous oxygen ($SpO_2$). According to an embodiment, the processor 120 may determine biometric information of the body of the user U based on a signal detected by the second optical sensor 360. For example, the processor 120 may determine at least one of a heart rate of the user U, a blood oxidation level of the user U, or a blood glucose level of the user U based on the signal obtained from the second optical sensor 360.

According to an embodiment, information detected by the first optical sensor 350 and/or the second optical sensor 360 may be transmitted to the ADC 230 through an amplifier 371 and/or a signal adjuster 372. The amplifier 371 may increase the signal detected by the first optical sensor 350 and/or the second optical sensor 360 at a constant rate. For example, the amplifier 371 may be transimpedance amplifiers. The signal adjuster 372 may adjust the output of the amplifier 371 to correspond to the input of the ADC 230. According to an embodiment, the processor 120 may control the signal adjuster 372.

According to an embodiment, the processor 120 may determine a hue of the discoloration member 330 using a machine learning algorithm. For example, the processor 120 may determine the hue of the discoloration member 330 using symbolic regression based on genetic optimization of a syntax tree math formula. According to an embodiment, the processor 120 may convert light generated by the light emitting module 340 into a temperature of the discoloration member 330 using low parameters (e.g., less than 10) retrieved by the symbolic regression.

According to an embodiment, the electronic device 101 may include a temperature sensor (not shown) disposed in the housing 310. The processor 120 may detect the temperature of the ear tip 320 and/or the discoloration member 330 using the temperature sensor when the electronic device 101 is being charged.

According to an embodiment, the electronic device 101 may include an oscillator for adjusting a signal transmitted to the light emitting unit driving circuit 344. According to one embodiment, a signal transmitted to the light emitting diode 341 may be tuned by an oscillator to form a sequential illumination.

According to an embodiment, the electronic device 101 may include an ambient light cancellation device for cancelling noise of the light reflected from the discoloration member 330 and/or the user U. According to an embodiment, the oscillator may adjust a signal applied to the ambient light cancellation device (not shown). According to an embodiment, the ADC 230 may store the signal transmitted from the ambient light cancellation device in a data register.

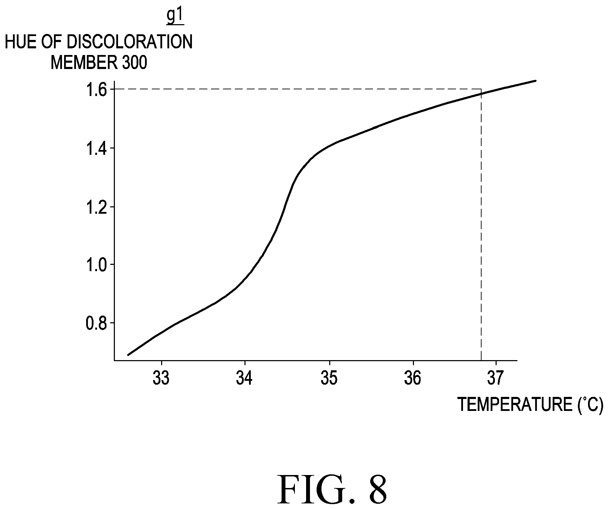

FIG. 8 is a view illustrating a change in the hue of a discoloration member that is changed based on a second light detected by the first optical sensor according to an embodiment of the disclosure. For example, the horizontal axis of a first graph g1 of FIG. 8 may represent temperature (unit: °C) of the discoloration member 300, and the vertical axis thereof may represent a hue of the discoloration member 300. The hue of the discoloration member 300 may be referred to as a hue angle (unit: rad) reflecting a ratio of red, green, and blue.

Referring to FIG. 8, the hue of the discoloration member 300 may be changed based on the temperature of the discoloration member 300. The configurations of the discoloration member 300 of FIG. 8 may be entirely or partially the same as those of the discoloration member 330 of FIG. 4.

According to an embodiment, the processor (e.g., the processor 120 of FIG. 1) may determine the user's temperature based on information detected using the first optical sensor 350. According to an embodiment, the hue of the discoloration member 330 may be changed based on the temperature, and the processor 120 may determine the user's temperature based on the hue of light (e.g., the second light L2 of FIG. 5) reflected from the discoloration member 330. For example, the first optical sensor (e.g., the first optical sensor 350 of FIG. 4) may detect red light, green light, and blue light of the second light L2, and the processor 120 may determine the temperature of the discoloration member 330 and/or the temperature of the user based on a ratio of red, green, and blue of the second light L2.

According to an embodiment, the processor 120 may determine the temperature of the user's body based on the temperature of the discoloration member 330. For example, the processor 120 may determine the temperature of the user's body (e.g., an ear) by calibrating the temperature of the discoloration member 330.

According to an embodiment, a memory (e.g., the memory 130 of FIG. 1) may store hue information corresponding to the temperature, and the processor 120 may determine the temperature of the discoloration member 330 and/or the temperature of the user based on the hue information.

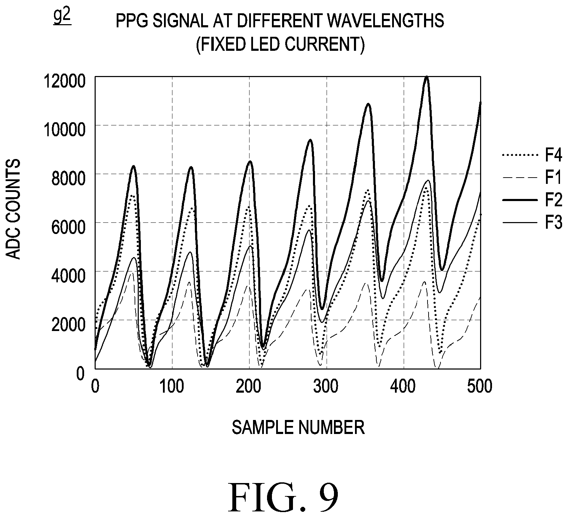

FIG. 9 is a view illustrating a value of photoplethysmography (PPG) that is changed based on a fourth light detected by the second optical sensor according to an embodiment of the disclosure. For example, the horizontal axis of a second graph g2 of FIG. 9 may be interpreted as time, and the vertical axis thereof may be interpreted as the amount of light detected by the second optical sensor (e.g.: the second optical sensor 360 of FIG. 5).

Referring to FIG. 9, the processor (e.g., the processor 120 of FIG. 1) may determine the user's body information based on light detected by the second optical sensor 360. According to an embodiment, the processor 120 may determine the user's photoplethysmography (PPG) based on the information detected using the second optical sensor 360. For example, the light emitting module (e.g., the light emitting module 340 of FIG. 5) may emit a third light L3 (e.g., the third light L3 of FIG. 5) toward the user's body (e.g., an ear). The second optical sensor 360 may detect a part of the third light L3 as a fourth light L4 reflected from the user's body (e.g., the user's blood vessel). When a volume of the user's blood vessels changes due to the heartbeat, the processor 120 may determine the user's pulse based on the intensity of illumination of the fourth light L4 detected by the second optical sensor 360. According to an embodiment, the fourth light L4 may include light of various wavelengths. For example, the fourth light L4 may include red light F 1, green light F2, blue light F3, and/or light F4 having an infrared wavelength band. According to an embodiment (not shown), the processor 120 may determine saturation of percutaneous oxygen ($SpO_2$) of the user based on the information detected using the second optical sensor 360. For example, the processor 120 may determine the oxygen saturation of the user's arterial blood.

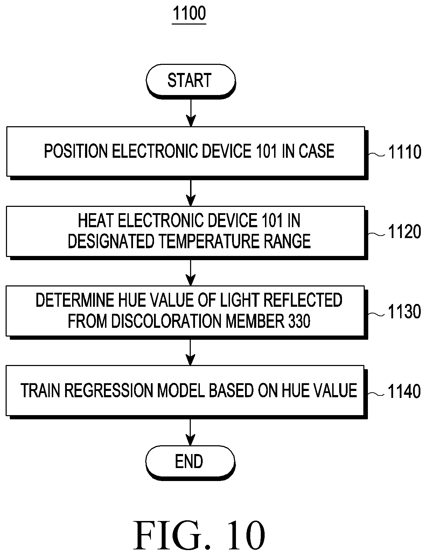

FIG. 10 is a block diagram illustrating an operation of an electronic device according to an embodiment of the disclosure.

Referring to FIG. 10, the regression model training 1100 of the electronic device may include operation 1110 of positioning the electronic device 101 in a case (not shown), operation 1120 of heating the electronic device 101 in a designated temperature range, operation 1130 of determining a hue value of the light reflected from the discoloration member 330, and operation 1140 of training a regression model based on the hue value. The configurations of the electronic device 101 and the discoloration member 330 of FIG. 10 may be entirely or partially the same as those of the electronic device 101 and the discoloration member 330 of FIG. 7.

According to an embodiment, in order to determine a temperature based on the hue (e.g., R, G, and B values) of the discoloration member 330 of the electronic device 101, calibration may be required. According to an embodiment, in operation 1110 of positioning the electronic device 101 in the case (not shown), the case may include a thermal sensor contacting the electronic device 101.

According to an embodiment, while the electronic device 101 is being charged, a coil used for charging may generate heat, and based on the generated heat, the electronic device 101 may be heated up to about 42°C. For example, operation 1120 of heating the electronic device 101 may be referred to as an operation of charging the electronic device 101. According to an embodiment, in the operation of charging the electronic device 101, in a case where the electronic device 101 is in a constant voltage state, the temperature of the electronic device 101 may decrease and the temperature of the discoloration member 330 may also decrease. According to an embodiment, when the user charges the electronic device 101, a calibration for measuring the temperature of the electronic device 101 may be performed.

According to an embodiment, while the electronic device 101 is being charged in the case, operation 1130 of storing the hue value of light reflected from the discoloration member 330 may be performed. For example, the memory (e.g., the memory 130 of FIG. 1) of the electronic device 101 may store the temperature of the electronic device 101 and the hue value (e.g., a red, green, or blue value) of light corresponding to the temperature. According to an embodiment, operation 1140 of training the regression model based on the hue value may be trained based on a mapping function using a nonlinear regression model.

The hue value of the electronic device 101 may be converted into a hue color space. The hue color space H may be defined using Equation 1 and Equation 2 below.

【Equation 1】

$$H' = undefined (if C = 0), H' = \frac{G\text{-}B}{C} \bmod 6 (if M = R), H' = \frac{B\text{-}R}{C} + 2 (if M = G), H' = \frac{R\text{-}G}{C} + 4 (if M = B)$$

【Equation 2】

$$H = 60^{\circ} \times H'$$

In Equation 1 above, M=max(R, G, B), m=min(R,G,B), and C=range(R,G,B)=M-m. For example, R, G, and B may indicate the amount and/or energy of red light, green light, or blue light. It may be interpreted that max(R, G, B) indicates an amount of light of a hue having the largest light amount among light amounts of red light, green light, or blue light. It may be interpreted that min(R,G,B) indicates the amount of light of a hue having the smallest amount of light among light amounts of red light, green light, or blue light.

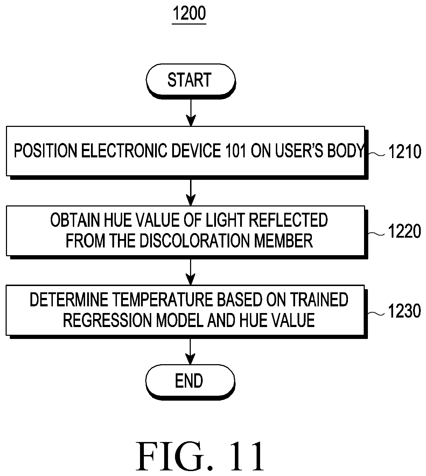

FIG. 11 is a flowchart illustrating an operation of an electronic device according to an embodiment of the disclosure.

Referring to FIG. 11, operation 1200 of the electronic device may include operation 1210 of positioning the electronic device 101 on the user's body, operation 1220 of obtaining a hue value of light reflected from the discoloration member 330, and operation 1230 of determining temperature based on the trained regression model and the hue value. The configurations of the electronic device 101 and the discoloration member 330 of FIG. 11 may be entirely or partially the same as those of the electronic device 101 and the discoloration member 330 of FIG. 7.

According to an embodiment, the operation performed when the electronic device 101 is positioned on the user's body (e.g., an ear) may be substantially the same as the operation performed when the electronic device 101 is positioned in the case. For example, the electronic device 101 may obtain a hue value of light reflected from the discoloration member 330. The hue value may be referred to as the hue color space (H).

According to an embodiment, the electronic device 101 may determine the temperature of the user's body and/or the electronic device 101 (e.g., the discoloration member 330) based on the hue value. For example, the electronic device 101 (e.g., the processor 120 of FIG. 7) may determine the temperature of the electronic device 101 (e.g., the discoloration member 330) and/or user's body (e.g., ears) based on the hue value and the regression model trained in the calibration step. For example, a value obtained from red light, green light, or blue light may be converted into a hue value, and the hue value may be converted into temperature using the regression model obtained in the calibration step.

According to various embodiments of the disclosure, an electronic device (e.g., the electronic device 101 of FIG. 3) may include a housing (e.g., the housing 310 of FIG. 3) including a protrusion (e.g., the protrusion 314 of FIG. 4), an ear tip (e.g., the ear tip 320 of FIG. 3) configured to be connected to the protrusion, a discoloration member (e.g., the discoloration member of FIG. 4) disposed on an inner surface (e.g., the inner surface 320a of FIG. 4) of the ear tip and configured to change a hue based on temperature, a light emitting module (e.g., the light emitting module 340 of FIG. 4) disposed in the housing and configured to emit light toward the discoloration member, a first optical sensor (e.g., the first optical sensor 350 of FIG. 5) disposed in the housing and configured to detect light reflected from the discoloration member, and a processor (e.g., the processor 120 of FIG. 1) configured to determine a user's temperature based on a hue detected using the first optical sensor.

According to an embodiment, the discoloration member may include thermochromic liquid crystals (TLC).

According to an embodiment, the discoloration member may comprise cholesteryl oleyl carbonate, cholesteryl nonanoate, and/or cholesteryl benzoate.

According to an embodiment, the discoloration member may comprise the cholesteryl oleyl carbonate, the cholesteryl nonanoate, and the cholesteryl benzoate in a ratio of about 3:6:1, respectively.

According to an embodiment, the discoloration member may be configured to change hue within a designated temperature range.

According to an embodiment, the designated temperature range may be approximately 37 to 40 degrees Celsius. According to an embodiment, the electronic device may further include a second optical sensor (e.g., the second optical sensor 360 of FIG. 5) disposed in the housing and configured to detect light reflected from a user's body.

According to an embodiment, the processor may perform photoplethysmography (PPG) of the user based on a signal detected using the second optical sensor.

According to an embodiment, the first optical sensor may be spaced apart from the second optical sensor.

According to an embodiment, the first optical sensor and the second optical sensor may be configured to detect light generated by the same light emitting module.

According to an embodiment, the light emitting module may include a light emitting diode (e.g., the light emitting diode 341 of FIG. 5) configured to emit light having at least two distinguishable wavelengths.

According to an embodiment, the light emitting module may include a first light path (e.g., the first light path 342 of FIG. 5) that is connected to the light emitting diode and configured to transmit light toward the discoloration member.

According to an embodiment, the light emitting module may include a second light path (e.g., the second optical path 343 of FIG. 5) that is spaced apart from the first light path and configured to transmit light reflected from the discoloration member to the first optical sensor.

According to an embodiment, each of the first light path and the second light path may comprise at least one of an optical cable or a waveguide.

According to an embodiment, the discoloration member may be coated on the inner surface of the ear tip.

According to an embodiment, the processor may be configured to determine at least one of a heart rate, a blood oxidation level, or a blood glucose level based on a signal obtained from the second optical sensor.

According to an embodiment, the processor may be configured to determine a temperature of the discoloration member using symbolic regression.

According to an embodiment, the ear tip may include silicone.

According to an embodiment, the first optical sensor may be disposed on the protrusion.

According to an embodiment, the electronic device may further include a temperature sensor disposed in the housing, wherein the processor may be configured to determine a temperature of the discoloration member using the temperature sensor when the electronic device is being charged, and to calibrate a temperature of the discoloration member based on the temperature of the discoloration member detected by the temperature sensor, and the hue of the discoloration member.

According to an embodiment, the electronic device may further include a speaker disposed in the housing; and a battery disposed in the housing and configured to supply power to the processor and the speaker.

According to an embodiment, the electronic device may comprise an ambient light cancellation device configured to cancel noise of light reflected from the discoloration member and/or the user.

While the disclosure has been shown and described with reference to various embodiments thereof, it will be understood by those skilled in the art that various changes in form and details may be made therein without departing from the spirit and scope of the disclosure as defined by the appended claims and their equivalents.

**Claims**

1. An electronic device comprising:

   a housing including a protrusion;
   an ear tip configured to be connected to the protrusion;
   a discoloration member disposed on an inner surface of the ear tip and configured to change hue based on temperature;
   a light emitting module disposed in the housing and configured to emit light toward the discoloration member;
   a first optical sensor disposed in the housing and configured to detect light reflected from the discoloration member; and
   a processor configured to determine a user's temperature based on a hue of light detected by the first optical sensor.

2. The electronic device of claim 1, wherein the discoloration member includes thermochromic liquid crystals.

3. The electronic device of claim 1, further comprising a second optical sensor disposed in the housing and configured to detect light reflected from a user's body.

4. The electronic device of claim 3, wherein the processor is configured to perform photoplethysmography of the user based on a signal detected using the second optical sensor.

5. The electronic device of claim 3, wherein the processor is configured to determine at least one of a heart rate, a blood oxidation level, or a blood glucose level based on a signal obtained from the second optical sensor.

6. The electronic device of claim 3, wherein the first optical sensor is disposed closer than the second optical sensor to the protrusion.

7. The electronic device of claim 3, wherein the first optical sensor and the second optical sensor are configured to detect light generated by a same light emitting module.

8. The electronic device of claim 1, wherein the light emitting module comprises a light emitting diode (LED) configured to emit light having at least two distinguishable wavelengths.

9. The electronic device of claim 8, wherein the light emitting module comprises a first light path connected to the light emitting diode and configured to transmit light toward the discoloration member.

10. The electronic device of claim 9, wherein the light emitting module comprises a second light path spaced apart from the first light path, the second light path being configured to transmit light reflected from the discoloration member to the first light path.

11. The electronic device of claim 1, wherein the discoloration member is coated on an inner surface of the ear tip.

12. The electronic device of claim 1, wherein the processor is configured to determine a temperature of the discoloration member using symbolic regression.

13. The electronic device of claim 1, wherein the ear tip includes silicone.

14. The electronic device of claim 1, further comprising:

   a temperature sensor disposed in the housing,

wherein the processor is configured to determine a temperature of the discoloration member by using the temperature sensor when the electronic device is being charged, and

wherein the processor is configured to calibrate a temperature of the discoloration member based on the temperature of the discoloration member detected by the temperature sensor, and a hue of the discoloration member.

15. The electronic device of claim 1, further comprising:

a speaker disposed in the housing; and
a battery disposed in the housing, the battery being configured to supply power to the processor and the speaker.

FIG. 1

200

### 170

**AUDIO MODULE**

| 210 | 270 |
|---|---|
| AUDIO INPUT INTERFACE | AUDIO OUTPUT INTERFACE |

| 220 | 260 |
|---|---|
| AUDIO INPUT MIXER | AUDIO OUTPUT MIXER |

| 230 | 250 |
|---|---|
| ADC | DAC |

240

AUDIO SIGNAL PROCESSOR

# FIG. 2

FIG. 3

FIG. 4

FIG. 5

FIG. 6

FIG. 7

EP 4 432 699 A1

g1

HUE OF DISCOLORATION
MEMBER 300

TEMPERATURE (°C)

FIG. 8

g2   PPG SIGNAL AT DIFFERENT WAVELENGTHS
(FIXED LED CURRENT)

FIG. 9

1100

```
        ┌─────────┐
        │  START  │
        └─────────┘
             │
             ▼
┌──────────────────────────────────────┐
│  POSITION ELECTRONIC DEVICE 101 IN CASE │ ～ 1110
└──────────────────────────────────────┘
             │
             ▼
┌──────────────────────────────────────┐
│       HEAT ELECTRONIC DEVICE 101 IN    │ ～ 1120
│      DESIGNATED TEMPERATURE RANGE      │
└──────────────────────────────────────┘
             │
             ▼
┌──────────────────────────────────────┐
│  DETERMINE HUE VALUE OF LIGHT REFLECTED │ ～ 1130
│    FROM DISCOLORATION MEMBER 330       │
└──────────────────────────────────────┘
             │
             ▼
┌──────────────────────────────────────┐
│  TRAIN REGRESSION MODEL BASED ON HUE VALUE │ ～ 1140
└──────────────────────────────────────┘
             │
             ▼
        ┌─────────┐
        │   END   │
        └─────────┘
```

# FIG. 10

1200

START

POSITION ELECTRONIC DEVICE 101 ON USER'S BODY ~ 1210

OBTAIN HUE VALUE OF LIGHT REFLECTED
FROM THE DISCOLORATION MEMBER ~ 1220

DETERMINE TEMPERATURE BASED ON TRAINED
REGRESSION MODEL AND HUE VALUE ~ 1230

END

FIG. 11

<div align="center">

**INTERNATIONAL SEARCH REPORT**

</div>

| | International application No. |
|---|---|
| | **PCT/KR2023/001674** |

### A. CLASSIFICATION OF SUBJECT MATTER

**H04R 1/10**(2006.01)i; **H05B 33/12**(2006.01)i; **G02F 1/15**(2006.01)i; **G01K 11/12**(2006.01)i; **G01J 5/60**(2006.01)i; **A61B 5/024**(2006.01)i; **A61B 5/1455**(2006.01)i; **A61B 5/145**(2006.01)i; **A61B 5/00**(2006.01)i

According to International Patent Classification (IPC) or to both national classification and IPC

### B. FIELDS SEARCHED

Minimum documentation searched (classification system followed by classification symbols)

H04R 1/10(2006.01); A41D 13/05(2006.01); A61B 5/00(2006.01); A61B 5/01(2006.01); A61B 5/0205(2006.01); H04R 31/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 변색(discolor), 이어팁(ear tip), 빛(light), 센서(sensor), 온도(temperature), 반사(reflection)

### C. DOCUMENTS CONSIDERED TO BE RELEVANT

| Category* | Citation of document, with indication, where appropriate, of the relevant passages | Relevant to claim No. |
|---|---|---|
| Y | KR 10-2286970 B1 (UNITEL ELECTRONICS CO., LTD.) 06 August 2021 (2021-08-06)<br>   See paragraphs [0016]-[0023], claim 1 and figures 1-2. | 1-15 |
| Y | US 2014-0180039 A1 (VALENCELL, INC.) 26 June 2014 (2014-06-26)<br>   See paragraphs [0096]-[0135], claims 1-6 and figures 3-12B. | 1-15 |
| Y | CN 111314819 A (SHENZHEN LIDA INNOVATION TECHNOLOGY CO., LTD.) 19 June 2020 (2020-06-19)<br>   See claim 10. | 1-15 |
| Y | KR 10-2020-0141319 A (INDUSTRY-UNIVERSITY COOPERATION FOUNDATION SUNMOON UNIVERSITY) 18 December 2020 (2020-12-18)<br>   See paragraphs [0042]-[0046] and claims 1-3. | 14 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 May 2023** | **19 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2023/001674** |

**C.     DOCUMENTS CONSIDERED TO BE RELEVANT**

| Category* | Citation of document, with indication, where appropriate, of the relevant passages | Relevant to claim No. |
|---|---|---|
| A | KR 10-1562621 B1 (DAEGU GYEONGBUK INSTITUTE OF SCIENCE AND TECHNOLOGY) 22 October 2015 (2015-10-22)<br>    See entire document. | 1-15 |

Form PCT/ISA/210 (second sheet) (July 2022)

## INTERNATIONAL SEARCH REPORT
### Information on patent family members

International application No.

**PCT/KR2023/001674**

| Patent document cited in search report | | | Publication date (day/month/year) | Patent family member(s) | | | Publication date (day/month/year) |
|---|---|---|---|---|---|---|---|
| KR | 10-2286970 | B1 | 06 August 2021 | WO | 2021-215623 | A1 | 28 October 2021 |
| US | 2014-0180039 | A1 | 26 June 2014 | CN | 105379306 | A | 02 March 2016 |
| | | | | CN | 105379306 | B | 14 February 2020 |
| | | | | CN | 111464901 | A | 28 July 2020 |
| | | | | CN | 111464901 | B | 11 February 2022 |
| | | | | EP | 2400884 | A2 | 04 January 2012 |
| | | | | EP | 2400884 | A4 | 01 October 2014 |
| | | | | EP | 2400884 | B1 | 07 March 2018 |
| | | | | EP | 2405805 | A2 | 18 January 2012 |
| | | | | EP | 2405805 | A4 | 15 October 2014 |
| | | | | EP | 2932729 | A1 | 21 October 2015 |
| | | | | EP | 2932729 | A4 | 18 November 2015 |
| | | | | EP | 2932729 | B1 | 21 September 2016 |
| | | | | EP | 3127476 | A1 | 08 February 2017 |
| | | | | EP | 3128761 | A1 | 08 February 2017 |
| | | | | EP | 3128761 | B1 | 12 February 2020 |
| | | | | EP | 3357419 | A1 | 08 August 2018 |
| | | | | HK | 1210351 | A1 | 15 April 2016 |
| | | | | HK | 1232106 | A1 | 05 January 2018 |
| | | | | HK | 1254064 | A1 | 12 July 2019 |
| | | | | JP | 2012-518515 | A | 16 August 2012 |
| | | | | JP | 2015-231550 | A | 24 December 2015 |
| | | | | JP | 2016-013444 | A | 28 January 2016 |
| | | | | JP | 2017-159052 | A | 14 September 2017 |
| | | | | JP | 2018-108467 | A | 12 July 2018 |
| | | | | JP | 5789199 | B2 | 07 October 2015 |
| | | | | JP | 6126651 | B2 | 10 May 2017 |
| | | | | JP | 6153573 | B2 | 28 June 2017 |
| | | | | JP | 6309668 | B2 | 11 April 2018 |
| | | | | JP | 6745829 | B2 | 26 August 2020 |
| | | | | US | 08923941 | B2 | 30 December 2014 |
| | | | | US | 10076282 | B2 | 18 September 2018 |
| | | | | US | 10092245 | B2 | 09 October 2018 |
| | | | | US | 10448840 | B2 | 22 October 2019 |
| | | | | US | 10542893 | B2 | 28 January 2020 |
| | | | | US | 10716480 | B2 | 21 July 2020 |
| | | | | US | 10750954 | B2 | 25 August 2020 |
| | | | | US | 10842387 | B2 | 24 November 2020 |
| | | | | US | 10842389 | B2 | 24 November 2020 |
| | | | | US | 10898083 | B2 | 26 January 2021 |
| | | | | US | 10973415 | B2 | 13 April 2021 |
| | | | | US | 11026588 | B2 | 08 June 2021 |
| | | | | US | 11160460 | B2 | 02 November 2021 |
| | | | | US | 2010-0217098 | A1 | 26 August 2010 |
| | | | | US | 2010-0217099 | A1 | 26 August 2010 |
| | | | | US | 2010-0217100 | A1 | 26 August 2010 |
| | | | | US | 2010-0217102 | A1 | 26 August 2010 |
| | | | | US | 2013-0131519 | A1 | 23 May 2013 |
| | | | | US | 2014-0135596 | A1 | 15 May 2014 |
| | | | | US | 2014-0140567 | A1 | 22 May 2014 |

Form PCT/ISA/210 (patent family annex) (July 2022)

| INTERNATIONAL SEARCH REPORT | International application No. |
| Information on patent family members | PCT/KR2023/001674 |

| Patent document cited in search report | Publication date (day/month/year) | Patent family member(s) | | | Publication date (day/month/year) |
|---|---|---|---|---|---|
| | | US | 2014-0171755 | A1 | 19 June 2014 |
| | | US | 2014-0171762 | A1 | 19 June 2014 |
| | | US | 2014-0243620 | A1 | 28 August 2014 |
| | | US | 2014-0249381 | A1 | 04 September 2014 |
| | | US | 2014-0288394 | A1 | 25 September 2014 |
| | | US | 2014-0288395 | A1 | 25 September 2014 |
| | | US | 2014-0323830 | A1 | 30 October 2014 |
| | | US | 2015-0032009 | A1 | 29 January 2015 |
| | | US | 2015-0073236 | A1 | 12 March 2015 |
| | | US | 2015-0080741 | A1 | 19 March 2015 |
| | | US | 2015-0105633 | A1 | 16 April 2015 |
| | | US | 2015-0119657 | A1 | 30 April 2015 |
| | | US | 2015-0126824 | A1 | 07 May 2015 |
| | | US | 2015-0131837 | A1 | 14 May 2015 |
| | | US | 2015-0157222 | A1 | 11 June 2015 |
| | | US | 2015-0289818 | A1 | 15 October 2015 |
| | | US | 2015-0342467 | A1 | 03 December 2015 |
| | | US | 2016-0128637 | A1 | 12 May 2016 |
| | | US | 2017-0188851 | A1 | 06 July 2017 |
| | | US | 2018-0192950 | A1 | 12 July 2018 |
| | | US | 2018-0228435 | A1 | 16 August 2018 |
| | | US | 2018-0296165 | A1 | 18 October 2018 |
| | | US | 2018-0296166 | A1 | 18 October 2018 |
| | | US | 2018-0303430 | A1 | 25 October 2018 |
| | | US | 2019-0000396 | A1 | 03 January 2019 |
| | | US | 2019-0008460 | A1 | 10 January 2019 |
| | | US | 2019-0082974 | A1 | 21 March 2019 |
| | | US | 2019-0099130 | A1 | 04 April 2019 |
| | | US | 2021-0145290 | A1 | 20 May 2021 |
| | | US | 2021-0393146 | A1 | 23 December 2021 |
| | | US | 8647270 | B2 | 11 February 2014 |
| | | US | 8700111 | B2 | 15 April 2014 |
| | | US | 8788002 | B2 | 22 July 2014 |
| | | US | 8886269 | B2 | 11 November 2014 |
| | | US | 8929965 | B2 | 06 January 2015 |
| | | US | 8929966 | B2 | 06 January 2015 |
| | | US | 8934952 | B2 | 13 January 2015 |
| | | US | 8942776 | B2 | 27 January 2015 |
| | | US | 8961415 | B2 | 24 February 2015 |
| | | US | 8989830 | B2 | 24 March 2015 |
| | | US | 9131312 | B2 | 08 September 2015 |
| | | US | 9289135 | B2 | 22 March 2016 |
| | | US | 9289175 | B2 | 22 March 2016 |
| | | US | 9301696 | B2 | 05 April 2016 |
| | | US | 9314167 | B2 | 19 April 2016 |
| | | US | 9750462 | B2 | 05 September 2017 |
| | | US | 9955919 | B2 | 01 May 2018 |
| | | WO | 2010-098912 | A2 | 02 September 2010 |
| | | WO | 2010-098912 | A3 | 18 November 2010 |
| | | WO | 2010-098915 | A1 | 02 September 2010 |

Form PCT/ISA/210 (patent family annex) (July 2022)

## INTERNATIONAL SEARCH REPORT
### Information on patent family members

International application No.

**PCT/KR2023/001674**

| Patent document cited in search report | | | Publication date (day/month/year) | Patent family member(s) | | | Publication date (day/month/year) |
|---|---|---|---|---|---|---|---|
| | | | | WO | 2010-099066 | A2 | 02 September 2010 |
| | | | | WO | 2010-099066 | A3 | 18 November 2010 |
| | | | | WO | 2010-099190 | A2 | 02 September 2010 |
| | | | | WO | 2010-099190 | A3 | 06 January 2011 |
| | | | | WO | 2014-092932 | A1 | 19 June 2014 |
| CN | 111314819 | A | 19 June 2020 | None | | | |
| KR | 10-2020-0141319 | A | 18 December 2020 | None | | | |
| KR | 10-1562621 | B1 | 22 October 2015 | None | | | |

Form PCT/ISA/210 (patent family annex) (July 2022)